# EUROPEAN PATENT APPLICATION

(11) **EP 1 886 705 A1**
(43) Date of publication of application: **13.02.2008**
(21) Application number: 07253042.1
(22) Date of filing: 02.08.2007
(51) Int. Cl.: A61M 1/36

(54) **Arteriovenous shunt**

(30) Priority: 10.08.2006 US 502641
(71) Applicant: Cordis Corporation, Miami Lakes, FL 33014 (US)
(72) Inventor: Kopia, Gregory A., Hillsborough NJ 08844 (US)
(74) Representative: Belcher, Simon James

(57) **Abstract**

Arteriovenous grafts are utilized as connections between arteries and veins so that blood may be withdrawn and/or reintroduced. A number of pressure reducing graft configurations may be utilized to lower the static pressure on the venous side to a level closer to that on the arterial side.

In one embodiment, an arteriovenous shunt comprises a member having an internal diameter configured to produce a pressure drop across the length of the member when one end of the member is connected to an artery and another end of the member is connected to a vein.

## Description

The present invention relates to arteriovenous shunts, and more particularly to arteriovenous shunts configured to reduce the pressure on the venous side of the shunt.

There are a number of types of treatments in which blood is removed from a patient and passed through an extracorporeal blood circuit. Such treatments involve, for example, hemodialysis, hemofiltration, hemodiafiltration, plasmapheresis, blood component separation and oxygenation. Typically, the blood is removed from a blood vessel at an access site and returned to either the same blood vessel or at another location in the body.

In hemodialysis and similar treatments, an access site is surgically created in the nature of a fistula, i.e. passageway. Blood is removed from the fistula via an arterial needle and blood is returned to the fistula via a venous needle.

A common method of generating a permanent access site having the capability of providing a high blood flow and being operative during several years and even tens of years is the provision of an arteriovenous fistula. It is produced by operatively connecting the radial artery to the cephalic vein at the level of the forearm. The venous limb of the fistula thickens during the course of several months, thereby permitting repeated insertion of dialysis needles.

If a patient has small veins that will not develop into a workable fistula, vascular access may be achieved through a biological or synthetic tube implanted under the skin. The tube becomes an artificial vein that may be utilized repeatedly for needle placement and blood access. Since a graft does not need to develop as does a fistula, the graft may be utilized much more quickly. While grafts may tend to have more issues with clotting and/or infection as compared with fistulas, well cared for grafts may last several years.

An alternative to the arteriovenous fistula is the arteriovenous graft, in which a connection is generated from, for example, the radial artery at the wrist to the basilic vein. The connection may be made with a substantially tubular graft constructed from an autogenous saphenous vein or from a non-biological material such as polytetrafluorethylene. In this configuration, the needles are inserted into the graft rather than the fistula.

Other alternatives include the use of a dual-lumen catheter surgically implanted into one of the larger veins and/or a no-needle arteriovenous graft comprising a T-tube linked to a standard polytetrafluorethylene graft. In this alternative, the T-tube is implanted in the skin and vascular access is obtained either by unscrewing a plastic plug or by puncturing a septum of the T-tube with a needle. Other methods of obtaining access are known in the medical arts.

During hemodialysis, it is desirable to obtain a constant blood flow rate in the range of 150 to 500 ml/min or even higher; accordingly, the access site should preferably be prepared for delivering these high flow rates. The blood flow in an arteriovenous fistula is often 800 ml/min or higher, thereby permitting delivery of a blood flow rate in the desired range. However, in the absence of a sufficient forward blood flow, the extracorporeal circuit blood pump will take up some of the already treated blood entering the fistula via the venous needle leading to poor treatment results.

The most common cause of poor flow in arteriovenous fistulas is partial obstruction of the venous limb due to fibrosis secondary to multiple venipunctures. Moreover, stenosis may also cause a reduction of access flow.

Arteriovenous grafts or shunts may also develop issues with respect to blood flow. Arteriovenous grafts or shunts, over time, may develop a thick, robust neointima especially on the venous side, which may in turn gradually occlude the vessel to a point where thrombosis ultimately occurs.

The present invention overcomes the disadvantages associated with the methods and devices as briefly described above.

In accordance with one embodiment, the present invention is directed to an arteriovenous shunt comprising a member having an internal diameter configured to produce a pressure drop across the length of the member when one end of the member is connected to an artery and another end of the member is connected to a vein.

In accordance with another embodiment, the present invention is directed to an arteriovenous shunt comprising a tapered elongate structure having a first end with a first diameter and a second end with a second diameter, the first diameter being greater than the second diameter, wherein the first end is configured for attachment to an artery and the second end is configured for attachment to a vein.

In accordance with yet another embodiment, the present invention is directed to an arteriovenous shunt comprising an elongate structure having a first section with a first diameter, a second section with a second diameter and a third section with a third diameter positioned between the first and second sections, the first and second diameters being substantially equal and greater than the third diameter, wherein the first section is configured for attachment to an artery and the second section is configured for attachment to a vein.

In accordance with yet another embodiment, the present invention is directed to an arteriovenous shunt comprising an elongate structure having first and second ends, the first end being configured for attachment to an artery and the second end being configured for attachment to a vein, and a pressure reduction device mounted within the elongate structure and configured to lower the static blood pressure within the elongate structure such that the static pressure at the second end is lower than at the first end.

The present invention is directed to an arteriovenous graft or shunt. Arteriovenous grafts are typically utilized to create shunts between arteries and veins for the removal and return of blood in patients undergoing extracorporeal blood treatment or repeated blood removal and/or return. Needles may be inserted into the graft rather than the patient's arteries and/or veins.

Since connecting the arterial circulation to the venous circulation with an arteriovenous graft exposes the venous side to supraphysiologic pressure, it has been hypothesized that the robust neointima that may form on the venous side is an adaptive mechanism. Accordingly, the present invention utilizes various means to reduce the static blood pressure across the short distance of the graft so as to prevent adaptive neointimal formation.

The present invention is directed to improved designs in arteriovenous grafts. Arteriovenous grafts may be utilized to create shunts between arteries and veins to create areas of withdrawal and readmission of blood in patients undergoing hemodialysis and/or other methods of extracorporeal blood treatments. By utilizing an arteriovenous shunt, needles may be inserted into the graft to obtain blood for the dialysis process rather than to repeatedly puncture a patient's arteries and veins. However, arteriovenous grafts, may over time, develop a thick, robust neointima that may gradually occlude the vessel to a point where thrombosis ultimately occurs. This neointima is more prevalent on the venous side. This phenomenon causes the need for a replacement of the arteriovenous graft.

Since connecting the arterial circulation to the venous circulation with an arteriovenous graft exposes the venous side to supraphysiologic pressure, it has been hypothesized that the robust neointima that forms is an adaptive mechanism. Accordingly, one potential means of limiting venous neointima formation might be to limit some portion of the arteriovenous graft diameter or some other design feature to produce a pressure drop across the length of the graft. This may permit the venous side of the arteriovenous graft to experience a lower pressure thereby potentially lessening adaptive neointimal formation. These design modifications are preferably positioned so as not to interfere with the needle placement utilized for the withdrawal and return of blood during dialysis.

The foregoing and other features and advantages of the invention will be apparent from the following more particular description of preferred embodiments of the invention as illustrated in the accompanying drawings.

Embodiments of the invention will now be described by way of example with reference to the accompanying drawings, in which:
Figure 1 is a diagrammatic representation of a first exemplary arteriovenous graft in accordance with the present invention.
Figure 2 is a diagrammatic representation of a second exemplary arteriovenous graft in accordance with the present invention.
Figure 3 is a diagrammatic representation of a third exemplary arteriovenous graft in accordance with the present invention.

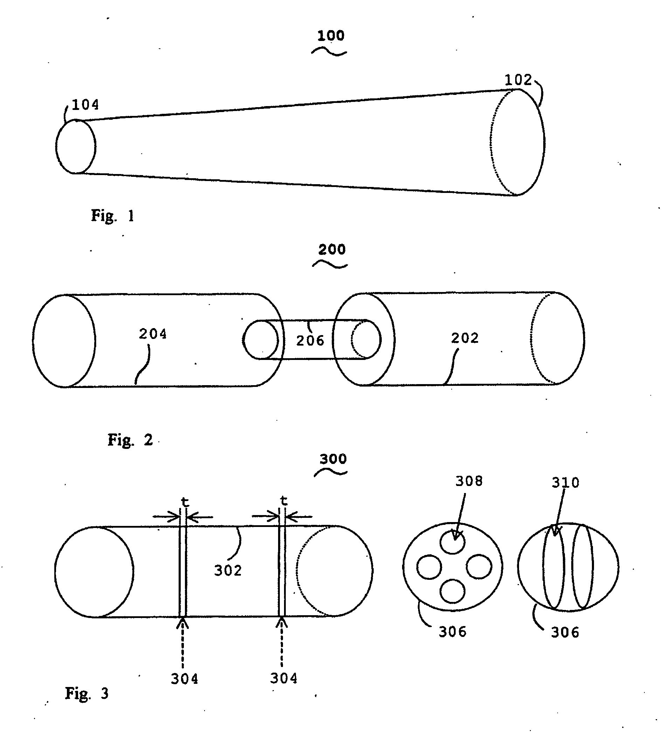

Referring to Figure 1, there is illustrated a first exemplary embodiment of an arteriovenous graft in accordance with the present invention. The arteriovenous graft 100 is preferably made from polytetrafluoroethylene; however, it is not intended that the invention be limited by the material from which the graft is constructed. It is contemplated that the graft may advantageously be made from a wide variety of biocompatible materials, now known or later developed, having the desirable physical characteristics of flexibility and strength.

The arteriovenous graft 100 comprises a first end 102 adapted and/or configured for attachment to an artery and a second end 104 adapted and/or configured for attachment to a vein. As illustrated, the arteriovenous graft 100 has a larger diameter at its first end 102 that continuously decreases to a smaller diameter at its second end 104 to form a frustum of a right circular cone. According to Bernoulli's Principle, as a fluid travels through a conduit that has a changing diameter, the velocity and pressure of the fluid also changes. More specifically, Bernoulli's Principle indicates that as the velocity of a fluid increases, the static pressure within the fluid decreases. Accordingly, in the exemplary embodiment illustrated in Figure 1, as the blood flows from the first end 102 to the second end 104, the velocity of the blood increases and the pressure within the blood decreases. Therefore, this design results in the venous side seeing a lower pressure thereby potentially reducing potential neointima formation.

Referring now to Figure 2, there is illustrated a second exemplary embodiment of an arteriovenous graft in accordance with the present invention. As described above, the arteriovenous graft 200 is preferably made from polytetrafluoroethylene, but may be made from any other suitable biocompatible material exhibiting flexibility and strength. The exemplary arteriovenous graft 200 comprises a first section 202 adapted and/or configured for attachment to an artery, a second section 204 adapted and/or configured for attachment to a vein, and a third section 206 positioned between the first section 202 and the second section 204. The first and second sections 202 and 204 are preferably configured as right circular cylinders having substantially equal diameters. The third section 206 also is preferably configured as a right circular cylinder but with a smaller diameter than the first and second sections 202 and 204. This smaller diameter third section 206 creates a pressure drop within the blood that results in the venous side seeing a lower pressure, thereby reducing potential neointima growth. The third section 206 essentially acts in a manner analogous to that of a resistor in a single resistive circuit. In other words, as there is a voltage drop across the resistor, there is a pressure drop across the flow restricted third section 206. In addition, similarly to the electric circuit, once there is a pressure drop, there can be no increase in pressure in the second section 204 without adding energy to the system.

Figure 3 illustrates yet another exemplary embodiment of an arteriovenous graft in accordance with the present invention. In this exemplary embodiment, the arteriovenous graft 300 comprises a right circular cylinder 302 and at least one flow restrictor 304 positionable within the right circular cylinder 302 at a predetermined location. In one exemplary embodiment, the flow resistor 304 comprises a disc 306 having a diameter substantially equal to the inside diameter of the right circular cylinder 302, a thickness t, and a plurality of openings or holes 308, each having a diameter significantly less than the inner diameter of the right circular cylinder 302. As the blood flows through the holes 308, the pressure in the blood decreases. In fact, as the thickness t of the disc 306 increases, the resistance to flow increases, thereby reducing the pressure further. It is important to note that the number of holes or openings 308 may vary in size, shape and number as well as the thickness t to vary the pressure. For example, the disc 306 may comprises substantially oval shaped openings 310. Once again, the decrease in pressure results in the venous side seeing a lower pressure.

As may be seen from above, any design that results in a pressure drop from the arterial end to the venous end may be suitable to control the formation of neointima. Also, the specific shapes and configurations are for illustrative purposes only as those shapes and designs may vary. In addition to these design features, therapeutic agents may be utilized to help prevent or slow the formation of neointima on the venous side. For example, a rapamycin alone or in combination with heparin, may be affixed to the arteriovenous graft directly or via a polymeric coating to inhibit neointimal growth.

While exemplary embodiments of arteriovenous grafts are described herein, it is important to note that the local delivery of drug/drug combinations may be utilized to treat a wide variety of conditions utilizing any number of medical devices, or to enhance the function and/or life of the device. For example, intraocular lenses, placed to restore vision after cataract surgery is often compromised by the formation of a secondary cataract. The latter is often a result of cellular overgrowth on the lens surface and can be potentially minimized by combining a drug or drugs with the device. Other medical devices which often fail due to tissue in-growth or accumulation of proteinaceous material in, on and around the device, such as dialysis grafts, colostomy bag attachment devices, ear drainage tubes, leads for pace makers and implantable defibrillators can also benefit from the device-drug combination approach. Devices that serve to improve the structure and function of tissue or organ, may also show benefits when combined with the appropriate agent or agents. For example, improved osteointegration of orthopedic devices to enhance stabilization of the implanted device could potentially be achieved by combining it with agents such as bone-morphogenic protein. Similarly other surgical devices, sutures, staples, anastomosis devices, vertebral disks, bone pins, suture anchors, hemostatic barriers, clamps, screws, plates, clips, vascular implants, tissue adhesives and sealants, tissue scaffolds, various types of dressings, bone substitutes, intraluminal devices, and vascular supports could also provide enhanced patient benefit using this drug-device combination approach. Perivascular wraps may be particularly advantageous, alone or in combination with other medical devices. The perivascular wraps may supply additional drugs to a treatment site. Essentially, any type of medical device may be coated in some fashion with a drug or drug combination which enhances treatment over use of the singular use of the device or pharmaceutical agent.

In addition to various medical devices, the coatings on these devices may be used to deliver therapeutic and pharmaceutic agents including: anti-proliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) 11_{b}/111ₐ inhibitors and vitronectin receptor antagonists; anti-proliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nirtosoureas (carmustine (BCNU) and analogs, streptozocin), trazenes - dacarbazinine (DTIC); anti-proliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6a-methylprednisolone, triamcinolone, betamethasone, and dexamethasone), non-steroidal agents (salicylic acid derivatives i.e. aspirin; paraaminophenol derivatives i.e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; antisense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

## Claims

1. An arteriovenous shunt comprising a member having an internal diameter configured to produce a pressure drop across the length of the member when one end of the member is connected to an artery and another end of the member is connected to a vein.

2. An arteriovenous shunt comprising a tapered elongate structure having a first end with a first diameter and a second end with a second diameter, the first diameter being greater than the second diameter, wherein the first end is configured for attachment to an artery and the second end is configured for attachment to a vein.

3. The arteriovenous shunt according to claim 2, wherein the tapered elongate structure comprises a biocompatible material.

4. The arteriovenous shunt according to claim 3, wherein the biocompatible material comprises polytetrafluoroethylene.

5. The arteriovenous shunt according to claim 2, further comprises at least one agent, in therapeutic dosage, affixed to at least a portion of the tapered elongate structure.

6. The arteriovenous shunt according to claim 3, wherein the at least one agent is directly affixed to a surface of the at least a portion of the tapered elongate structure.

7. The arteriovenous shunt according to claim 3, wherein the at least one agent is incorporated into a polymeric material and affixed to the at least a portion of the tapered elongate structure.

8. An arteriovenous shunt comprising an elongate structure having a first section with a first diameter, a second section with a second diameter and a third section with a third diameter positioned between the first and second sections, the first and second diameters being substantially equal and greater than the third diameter, wherein the first section is configured for attachment to an artery and the second section is configured for attachment to a vein.

9. The arteriovenous shunt according to claim 8, wherein the first, second and third sections comprise substantially tubular configurations.

10. An arteriovenous shunt comprising:
an elongate structure having first and second ends, the first end being configured for attachment to an artery and the second end being configured for attachment to a vein; and
a pressure reduction device mounted within the elongate structure and configured to lower the static blood pressure within the elongate structure such that the static pressure at the second end is lower than at the first end.

11. The arteriovenous shunt according to claim 10, wherein the pressure reduction device comprises a substantially disc shaped device having at least one opening.

12. The arteriovenous shunt according to claim 8 or 10, wherein the elongate structure comprises a biocompatible material.

13. The arteriovenous shunt according to claim 12, wherein the biocompatible material comprises polytetrafluoroethylene.

14. The arteriovenous shunt according to claim 8 or 10, further comprises at least one agent, in therapeutic dosage, affixed to at least a portion of the elongate structure.

15. The arteriovenous shunt according to claim 3 or 14, wherein the at least one agent comprises an anti-inflammatory agent.

16. The arteriovenous shunt according to claim 3 or 14, wherein the at least one agent comprises an anti-restenotic agent.

17. The arteriovenous shunt according to claim 3 or 14, wherein the at least one agent comprises an anti-thrombogenic agent.

18. The arteriovenous shunt according to claim 14, wherein the at least one agent is directly affixed to a surface of the at least a portion of the elongate structure.

19. The arteriovenous shunt according to claim 14, wherein the at least one agent is incorporated into a polymeric material and affixed to the at least a portion of the elongate structure.
